# EUROPEAN PATENT SPECIFICATION

(11) **EP 3 272 330 B1**
(45) Date of publication and mention of the grant of the patent: **18.08.2021**
(21) Application number: 17181452.8
(22) Date of filing: 14.07.2017
(51) Int. Cl.: A61K 8/67, A61Q 19/06

(54) **COMPOSITION AND COSMETIC METHOD FOR REDUCING CELLULITE BLEMISHES**
ZUSAMMENSETZUNG UND KOSMETISCHES VERFAHREN ZUR REDUKTION VON SCHÖNHEITSFEHLERN INFOLGE VON ZELLULITIS
COMPOSITION ET PROCÉDÉ COSMÉTIQUE POUR DIMINUER LES IMPERFECTIONS DE LA CELLULITE

(30) Priority: 18.07.2016 IT 201600075108
(43) Date of publication of application: 24.01.2018
(73) Proprietor: Italfarmacia S.r.l., 00155 Roma (IT)
(72) Inventor: Cavallo, Giovanni, I-00122 Ostia (RM) (IT); Marchetti, Mario, I-00161 Rome (IT); Marchetti, Marco, I-00167 Rome (IT); Marchetti, Massimiliano, I-00167 Roma (IT); Amuso, Domenico, I-41100 Modena (IT); Cavallo, Francesca, I-00122 Ostia (RM) (IT); De Lorenzo, Antonino, I-00133 Roma (IT); Di Renzo, Laura, I-00133 Roma (IT)
(74) Representative: Sarpi, Maurizio

(56) References cited:
- EP-A1- 0 717 983
- WO-A1-01/13865
- WO-A1-95/28084
- WO-A2-2011/156803
- US-A- 5 895 652
- US-A1- 2013 224 280
- DATABASE GNPD [Online] MINTEL; May 2016 (2016-05), Mead Johnson Nutrition: "Original flavoured step 3 formulated milk powder", XP002770477, Database accession no. 4007741
- DATABASE GNPD [Online] MINTEL; May 2003 (2003-05), Apotheker Scheller: "Aroma Vita Energy Lemongras Anti-Cellulite Oil", XP002770478, Database accession no. 206383
- DATABASE GNPD [Online] MINTEL; May 2008 (2008-05), Maria Galland: "Silhouette Body Sculpting Cream", XP002770479, Database accession no. 911919

## Description

### Field of application of the invention

The present invention relates to the field of cosmetic compositions for reducing of skin imperfections, in particular blemishes due to the presence of cellulite.

### Prior art

Cellulite is an aesthetic condition that sometimes may have also medical implications, caused by skin defects that determine the so-called "orange peel" effect. Cellulite is typically characterized by a condition of skin deterioration caused by breakdown of blood vessel integrity and loss of capillary network structure in the dermal and subcutaneous skin levels. Vascular deterioration results in a decrease in skin metabolism that obstructs protein synthesis and repair processes with loss of dermal tissue. The condition is further characterized by adipose cells that become swollen of lipids and by excess fluid retention in the skin and subcutaneous regions of the skin. Thus, individuals with cellulite tend to have a thicker layer of subcutaneous fat on the skin. In the more advanced stages of cellulite, in the skin around the deposits of fat proteins also deposits of reticular proteins, called sediments, blocking the fat cells begin to form. By further progression of the condition in the skin region hard nodules of fat cells and fatty aggregates surrounded by sects tend to form. All this leads to an extremely heterogeneous appearance of the skin surface from which the denomination "orange peel" derives. This feature is more pronounced in overweight subjects. Individuals presenting cellulite tend to have in the affected area thinner skin and dermis, decreased skin toning, and decreased cell renewal speeds.

Currently, the presence of cellulite tends to be eliminated, or limited, by the administration of xanthine, such as caffeine, theophylline and aminophylline. Xanthins act as a diuretic that removes water from fat cells, thus reducing the size of the fat cells. The effect of xanthine administration, however, is temporary and fat cells tend to swell as soon as the individual reintegrates the lost water.

European Patent EP1207840 discloses a composition for reducing or eliminating in a patient the appearance of cellulite comprising a sugar composition that in the patient is converted into a glycosaminoglycan in amount sufficient to imbue skin, an antioxidant agent sufficient to inhibit skin collagenase and elastase enzymes activity, at least one aminoacidic component in amount sufficient to facilitate skin thickening, and at least one transition metal in amount effective to bind collagen and elastic fibers, thereby contributing to a thickening effect of the skin. The so developed composition, which in a preferred embodiment also comprises at least one "fat burning" substance, to reduce fat absorption in the digestive tract or to prevent fat production, and/or at least a vascular dilator to improve fat loss, can be orally or topically administered. However, the use of certain glycosaminoglycans is not totally free of contraindications, especially for certain types of subjects.

Sometimes, in order to reduce and/or eliminate fat excess, localized adiposity and blemishes due to cellulite real medical or surgical treatments are used, but in this case the side effects may be particularly severe for the subject due to the invasiveness of the procedures.

More and more used, due to the lack of side effects, tolerability and great effectiveness, are the methods for reducing excess fat and cellular blemishes based on stimulation of apoptosis of adipocytes, or more generally on mechanisms of adipolysis/lipoclasis and lipolysis without induction of inflammation and necrosis.

Adipose tissue is functionally active, in 3-4 weeks it metabolizes and completely rebuilds all the triglycerides contained in its intradipocytic vacuole. Adipocyte triglycerides are subjected to active synthesis and lysis processes.

The normal volume of an adipocyte results from the continuous triglycerides synthesis and lysis process, under hormonal and genetic control. An unitary volume of adipose tissue can be formed either by a normal number of large cells (hypertrophic adiposity) or by a large number of normal size cells (hyperplastic adiposity). In the first case, to reduce and/or eliminate fat excess, local adiposity and blemishes due to cellulite it is necessary to reduce the adipocyte volume (lipolysis), while in the latter case it is necessary to reduce the number of cells by destroying the excess cells (lipoclasis).

In the field of adiposity treatment lipoclasis by apoptotic composition is carried out.

Apoptosis is a biological process inducing cell death without inflammatory manifestations.

Recent studies have shown that antioxidant agents, in small amounts, protect cell from oxidative damage, but at high concentrations perform pro-oxidative action, causing cell biological damage. In particular, high concentration of vitamin C induces cellular damage with cell destruction. Vitamin C in the presence of iron causes apoptosis of the cells wherein is injected.

In practice, by injecting into adipose tissue a sterile water solution with high concentrations of vitamin C and a few drops of iron and few anesthetic, adipose cells dissolving is produced, without inducing an inflammatory response. The biological process is characterized by a series of reactions leading to the formation of heterologous structures (apoptotic bodies) on the cell wall resulting in phagocytosis of the cell by macrophages. Cellular damage determines cytochrome C release from mitochondria and the activation of cell procaspases, their turning into caspase, and activating a series of chain reaction called caspase cascade. This leads to the activation of endonucleases destroying nuclear chromatin and of proteases that, by acting on the cell wall, release phosphatidylserine residues (apoptotic bodies) outward. Apoptotic bodies, recognized as non-macrophage-derived elements, stimulate cell phagocytosis.

The proposed solution for reducing and/or eliminating fat excess, localized adiposities and cellulite blemishes involving the apoptotic phenomenon activation, produces fragments of cells, apoptotic bodies that phagocytes, macrophages, and immune system cells rapidly embody and remove, before the cell contents can spill into the surrounding cells and cause damage. The mechanism has the advantage of not inducing an inflammatory response, however, the pro-oxidant action of vitamin C should be subjected to precise control and modulation so as not to cause undesirable cellular effects that may reach the necrosis. In the literature of the reference technical field for the same purpose various uses of ascorbic acid with varying concentrations in a fairly wide range and quite different are reported.

Gao Yan et al. have conducted a study wherein ascorbic acid was used to induce apoptotic damage and subsequent adipocytes decrease in concentration ranging from 0.24% to 0.12% (13.6-6.6 mM), whereas at a concentration of 0.06% (3.3 mM) is ineffective (Journal of Chinese Medicine Research, 2009, 9: 325-329).

Sakagami H. et al. have investigated the relationship between intensity of radical ascorbyl and the activity inducing apoptosis and indicated an ascorbic acid concentration range from 0.0176% to 0.176% (1-10mM) to induce an apoptotic effect (Anticancer Res. 1996 Sep-Oct; 16 (5A): 2635-44).

In order to treat localized adiposity by apoptosis of adipocytes Ceccarelli M. et al. use a 1% ascorbic acid solution in water (56.8 mM). According to such publication the apoptotic effect and eventual damage induced by ascorbic acid, also the osmotic one, would be enhanced in case the ascorbic acid solution is hypertonic.

It should not be overlooked that the uses referred to relate to vitamin C in the form of acid and not in the anionic form of its salt, suggesting that the acidity of the ascorbic acid solution is a further element contributing to the apoptotic elimination of adipocytes.

Since there are many factors involved in the induction of the apoptotic phenomenon, many factors need to be taken into account in order to optimize the effectiveness of a composition to be used in cosmetics treatments for the reduction of skin blemishes due to the presence of cellulite acting through adipolysis/adipoclasis without undesirable necrotic phenomena.

In fact,
(a) ascorbic acid concentration in the adipolytic solution has to enable adipocytes to be removed in a controlled and regular manner without causing necrosis;
(b) osmolality of the injected solution has not to cause or induce painful, inflammatory or necrotic phenomena, but ensure removal capacity of cellulite;
c) hypertonia of the ascorbic acid solution is important to induce cellulite removal, but a hypertonic solution may cause a slight local edema and/or inflammation such to promote inflammation or necrosis, vice versa a hypotonic/isotonic solution may not be effective;
d) the acidic pH of the solution containing ascorbic acid may cause a slight inflammation with the possibility of necrosis but a neutral pH solution may lose the ability to remove cellulite.

There is the need to improve the stability of the ascorbic acid solution to be used in cosmetic treatments to reduce the blemishes of cellulite. Therefore, there is a strong need for new compositions to be used in cosmetic treatments to reduce cellulite blemishes and deposit of localized fat based on a salt of ascorbic acid providing an *in vivo* apoptotic effect in the adipolysis/lipoclasis process, improving the phagocytes ability to remove cellular debris without inducing necrosis, being isotonic to avoid a possible induction of local inflammation, resulting in possible change from apoptotic phenomenon to necrotic phenomenon, keeping the efficacy of the treatment and the stability of the composition unchanged in an acceptable shelf life time.

### Summary of the invention

Object of the present invention is an aqueous isotonic and buffered composition with defined amounts of ascorbic acid salt (ascorbate) and ascorbyl palmitate as a lipolytic/lipoclasis agent, according to the enclosed claims.

Object of the invention is also the use of said composition comprising ascorbic acid salt (ascorbate) and ascorbyl palmitate as a lipolytic/lipoclasis agent to reduce cellulite blemishes in a cosmetic treatment as defined in the enclosed claims.

Another object of the invention is a kit that uses said composition for the cosmetic treatment of cellulite in subjects who need it for use by specialized operators in the field, such as aesthetic physicians.

### Brief description of the figures

Figure 1 shows the histological analysis of the adipose tissue of a patient who received treatment by administering a isotonic 0.24% sodium ascorbate, in phosphate buffer, composition.
Figure 2 shows histological analysis of the adipose tissue of a patient two hours after receiving treatment by administering a isotonic 0.24% sodium ascorbate, in phosphate buffer, composition.
Figure 3 shows the histological analysis of the adipose tissue of a patient to whom isotonic 0.12% sodium ascorbate, in phosphate buffer, composition, was administered.
Figure 4 shows the histological analysis of the adipose tissue of a patient who received treatment by administering the composition of 0.20% sodium ascorbate isotonic in phosphate buffer.
Figure 5 shows the histological analysis of the adipose tissue of a patient 7 days after treatment by administration of 0.20% sodium ascorbate, 0.020% ascorbyl palmitate composition.
Figure 6 shows the histology of a patient's adipose tissue 7 days after receiving treatment with 0.24% sodium ascorbate, 0.024% glycerol monooleate composition.
Figure 7 shows the histology of a patient's adipose tissue two hours after receiving treatment with 0.24% sodium ascorbate, 0.040% polysorbate 80 composition.
Figure 8 shows the histology of a patient's adipose tissue two days after receiving 0.24% sodium ascorbate, 0.040% polysorbate 80 composition.
Figure 9 shows the histological analysis of the adipose tissue of a patient to whom 0.24% sodium ascorbate, 0.020% ascorbyl palmitate composition, was administered.
Figure 10 shows the histological analysis of adipose tissue in a patient 5 days after administration of 0.24% sodium ascorbate, 0.020% ascorbyl palmitate composition.
Figure 11 shows the effect of treatment by administering 0.24% sodium ascorbate, 0.020% ascorbyl palmitate composition 10 days after administration.
Figure 12 shows the histological analysis of adipose tissue 15 days after administration of 0.24% sodium ascorbate, 0.020% ascorbyl palmitate composition.
Figure 13 shows the histology of adipose tissue 23 days after administration of 0.24% sodium ascorbate, 0.020% ascorbyl palmitate composition.
Figure 14 shows the histological analysis of adipose tissue with areas of evident lipoclasis 25 days after the first administration of 0.24% sodium ascorbate, 0.020% ascorbyl palmitate composition.

### Detailed description of the invention

It is already known to use either a salt of ascorbate or lipophilic derivatives to treat cellulite (WO 2011/156803 A2 (Transdel Pharmaceuticals Inc. (US) and W= 01/13865 A1 (Murad Howarde (US) and also to combine different forms of ascorbate (WO 95/28084 A1, (Metagenics Inc (US) EP 0 717 983 A1 (Unilever PLC) (GB), Unilever NV (NL), US 2013/224280 A1 (Toth Rita) (US) in product for the treatment of cellulite. Furthermore, from DATABASE GNPD, (Online), MINTEL, May 2016 (2016-05) Data base accession 206383, and MINTEL, May 2008 (2008-05), Database accession no.911919, there are known compositions for cellulite treatment which comnprise ascorbyl palmitate and ascorbic acid.

In order to develop a composition to be used in cosmetic treatments to reduce cellulite blemishes and localized fat accumulation, the Applicant has focused the research on the salty form of ascorbic acid since it is more stable in solution than its acidic form, less toxic, however finding still discrepancies in the efficacy of compositions comprising ascorbate salt at a concentration of 0.12% and 0.24%, with respect to compositions comprising a equal concentration of ascorbic acid as reported by Gao Yan et al. In particular, the Applicant found that a buffered isotonic composition comprising a salt of ascorbic acid (ascorbate) in a concentration of 0.12% by weight was ineffective, and a buffered isotonic composition comprising, in particular, 0.24% sodium ascorbate in weight resulted in necrosis; finally, a buffered isotonic composition of 0,20% ascorbic acid salt showed a low adipolytic ability, but not adequate for the actual removal of cellulite, and no toxicity. Unexpectedly, it has been found that the addition of a particular surfactant enables to enhance the lipolytic effect of the composition comprising 0.20% ascorbate by weight. The ability of various types of surfactants to determine the improvement of the lipolytic action of ascorbic acid salt has been assayed. Surprisingly, it has been found that lipophilic derivatives of ascorbic acid, particularly those substituted with palmitic acid and stearic acid, including, for example, ascorbyl palmitate, ascorbyl dipalmitate, ascorbyl tetraisopalmitate, ascorbyl stearate and tetraesildecyl ascorbate are the most effective. In particular, ascorbyl palmitate resulted to be particularly effective for this purpose and therefore, in a particularly preferred embodiment of the invention, the properties of the association of ascorbic acid salt and a lipophilic derivative of ascorbic acid, ascorbyl palmitate, are exploited.

The presence of 0.2-0.24% ascorbyl palmitate (10-12 mM) in isotonic solution of ascorbate determines an excellent adipolytic/lipoclasic effect without any inflammation or necrosis, adversely to observation of insufficient efficacy of a 0.2% ascorbate composition (10 mM) and of pronounced necrosis due to 0.24% sodium ascorbate (12 mM) solution.

Therefore, a first object of the invention is the association of a salt of ascorbic acid and a lipophilic derivative of ascorbic acid variedly substituted with palmitic acid or stearic acid, and its use as a lipolytic/lipoclasic agent.

According to the invention, the ascorbic acid salt in the association is a salt of a metal cation, preferably mono or bivalent, even more preferably a sodium, lithium, potassium, magnesium, calcium salt. In a particularly preferred embodiment, the salt is sodium ascorbate.

Among the lipophilic derivatives of ascorbic acid effective in the association according to the invention there is ascorbyl palmitate, which is an antioxidant agent obtained from the reaction between ascorbic acid (vitamin C) and palmitic acid (a fatty acid).

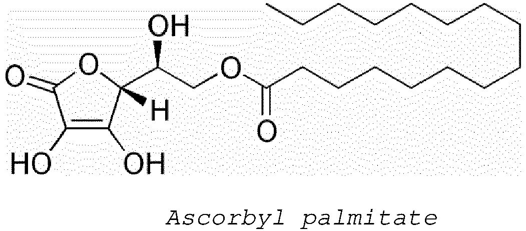

The strong interest of the industry in this molecule is related to that to vitamin C. In fact, the vitamin C molecule is very unstable and is among the most thermolable compounds. Because of its structural features, the vitamin C molecule is water soluble and has no affinity for environments where lipids or fats are present. By converting the ascorbic acid into ascorbyl palmitate, the molecule can come into contact with fat matrixes, thus greatly expanding its possible applications. In addition, the ascorbyl palmitate molecule is also more stable than free ascorbic acid. This feature allows the use thereof not only in the food and pharmaceutical industries, but also frequently the inclusion among the ingredients of cosmetic products.

According to the invention, in addition to the ascorbyl palmitate, other lipophilic derivatives, including ascorbyl dipalmitate, may be used in association with the ascorbic acid sodium salt;

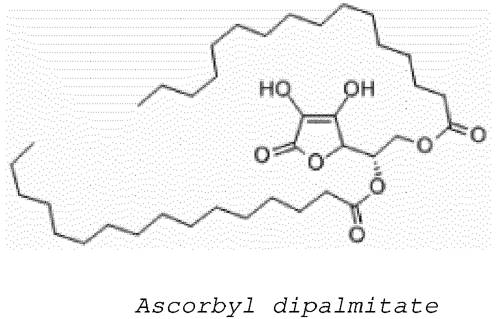

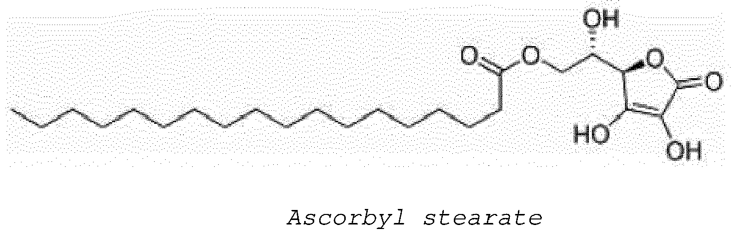

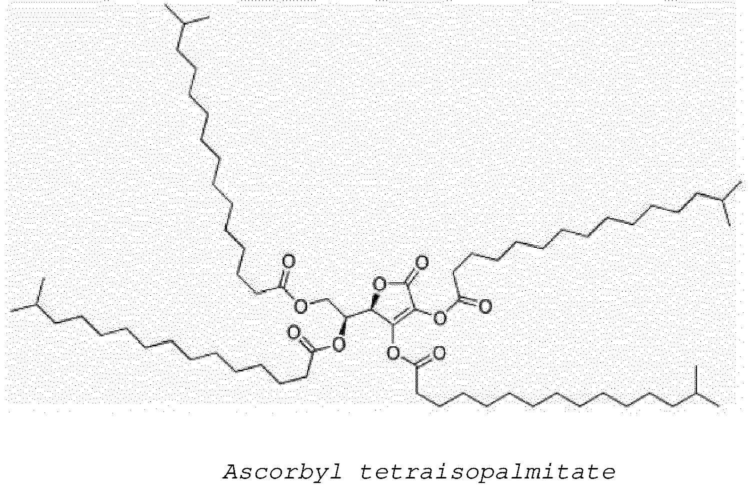

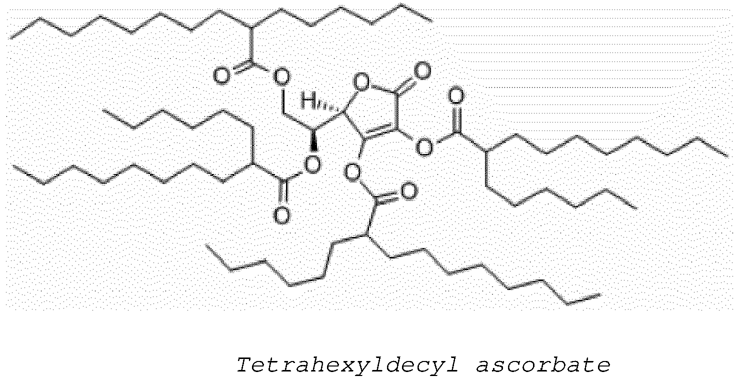

In the association according to the invention, the ascorbic acid lipophilic derivative is in acid or in salified form, and ascorbic acid and ascorbic acid lipophilic acid salt are respectively in the ratio of 10-15:1, preferably are in ratio of 12:1.

A further object of the invention is the composition having lipolytic action by activating the *in situ* apoptotic response, which comprises an ascorbic acid salt and a lipophilic derivative of ascorbic acid as ascorbyl palmitate.

The composition according to the invention is an aqueous, clear and colorless solution with pH between 7.0 and 7.4, preferably 7.14, and osmolality between 250 and 340 mOsm/Kg.

The pH is obtained by the presence in the composition of a buffering system able to maintain value in the range of 7.0 to 7.4, preferably 7.14. Various buffering systems known to the skilled in the art may be employed in the achievement of the present invention. However, in some preferred embodiments of the present invention, the monobasic/dibasic potassium phosphate system providing adequate buffering ability to maintain the pH value within the desired range is used.

The osmolality of the composition is ensured by the presence of an adequate amount of farmaceutically acceptable salt, preferably the composition comprises sodium chloride in amount suitable for determining an osmolality value of between 250 and 340 mOsm/Kg.

Thus, the lipolytic composition is a buffered and isotonic aqueous solution that in addition to ascorbic acid salt in a concentration varying between 0.10 and 0.30% p/p, preferably between 0.12 and 0.25% p/P, more preferably 0.24% w/w, and lipophilic derivative of ascorbic acid in a concentration between 0.005 and 0.05% w/w, preferably the concentration of the lipophilic derivative of ascorbic acid is between 0, 01 and 0.03% p/p, even more preferred the lipophilic derivative of ascorbic acid concentration is 0.02% p/p. The composition includes sodium chloride and a buffering system.

The composition according to the present invention may also comprise acceptable solvents, excipients, diluents and acceptable carriers which allow administration by injection into a subject that needs cosmetic treatment to reduce the blemishes of cellulite.

The formulation of the composition according to the invention in a particularly preferred embodiment thereof is presented below. Other aspects, advantages or modifications within the scope of the invention will be apparent to those skilled in the art pertaining to the invention and will in particular appear from the presentation of the examples and experimental data attached to this description.

A further object of the invention is the cosmetic treatment that allows to reduce or eliminate cellulite. The treatment involves administering subcutaneous injection to a subject of an effective amount of the above-described composition utilizing the pro-oxidizing properties of an ascorbic acid salt in the presence of a lipophilic derivative of ascorbic acid variedly substituted with palmitic acid or stearic acid, to perform a lipolytic action by activating the apoptotic process.

The expression "effective amount" refers to the amount of active ingredient(s) contained in the composition according to the invention which provides the technical effect of the reduction or elimination cellulite appearance and other skin defects related to the presence of cellulite.

In general, the cosmetic treatment according to the invention provides that a 5 ml dose of the composition according to the invention containing 0.24% by weight of ascorbic acid salt and 0.02% by weight of lipophilic derivative of ascorbic acid, which is substituted with palmitic acid or stearic acid, is administered by subcutaneous injection to treat a body surface area of 20 cm², preferably said dose is used to treat a body surface area of 10 cm².

The cosmetic treatment provides that administration is repeated after 15 or 21 days.

The skilled person can easily understand that the amount of composition to be administered, the frequency and the dosages, must take into account the severity of the condition to be treated. Dosage and frequency of administration vary according to age, body weight and individual response. Between two subsequent administrations, the area of the body wherein the injections are made in any case has to be subjected to careful and fine control to detect possible undesirable cytotoxic effects due to individual reactions.

Object of the invention is also a kit comprising:
a) at least one dark glass vial of 10 mL volume containing the mixture of freeze-dried powders composed of sterile ascorbic acid salt, phosphate buffer and sodium chloride, in amount necessary for the topical administration of one dose of the final reconstituted composition as above defined;
b) at least one vial of 5 ml volume containing 5 ml of sterile diluent comprising the lipophilic derivative of ascorbic acid substituted by palmitic acid or stearic acid, necessary to reconstitute the amount of mixture in (a) for the topical administration of one dose of the final reconstituted composition as above defined.

The invention will now be further described with reference to the examples describing in detail the preparation of the composition and its use.

### EXAMPLES

### Example 1 - Formulation of a preferred embodiment of the composition

| Ingredient | w/w % |
|---|---|
| Sodium ascorbate | 0.24 |
| Ascorbyl palmitate | 0.020 |
| Sodium chloride | 0.96 |
| Potassium dibasic phosphate (K₂HPO₄) | 0.0378 |
| Potassium monobasic phosphate (KH₂PO₄) | 0.010 |
| Distilled water | q.s. to 100. |

The formulation with the above percent composition is obtained when the sterile diluent solution containing the lipophilic derivative of ascorbic acid is added shortly before subcutaneous administration to the lyophilized powder mixture (sodium ascorbate, sodium chloride and buffer system).

### Example 2 - Identification of active formulations containing ascorbic acid salt.

In order to confirm the literature data, replace ascorbic acid with its salt and define the production process, five identical compositions in qualitative formulation differing only for the concentration of ascorbate have been prepared. Sodium salt was used in the experimentation. Below are the concentrations of sodium ascorbate compositions.
1) sterile, isotonic, buffered, 0.12% sodium ascorbate (6 mM) solution;
2) sterile, isotonic, buffered, 0.15% sodium ascorbate (7.5 mM) solution;
3) sterile, isotonic, buffered, 0.17% sodium ascorbate (8.5 mM) solution;
4) sterile, isotonic, buffered, 0.20% sodium ascorbate (10 mM) solution;
5) sterile, isotonic, buffered, 0.24% sodium ascorbate (12 mM) solution.

To ensure the stability of sodium ascorbate in the preparation of the compositions, the following procedure was carried out:
(a) preparation of the mixture containing the amount of sodium ascorbate necessary to achieve the desired concentration (0.12 to 0.24%) and the amount of sodium chloride necessary to obtain a concentration of 0.45%, lyophilized, sterile;
(b) preparation of the solvent solution containing the amount of sodium chloride (0.78%) required to reach the amount of the final composition and the sterile buffer system (buffered diluent);
(c) mixing of the mixture of sodium ascorbate and freeze-dried sodium chloride and buffered diluent solution thoroughly until complete dissolution of the lyophilized components so that the product is ready for use.

Step (a) Various solutions from 0.12 to 0.24% sodium ascorbate, 0.45% sodium chloride in water were sterilized by filtration with 0.22 µm filter, distributed in sterile, dark vials and subjected to lyophilization.

Step (b) The hypotonic diluent solution (0.78% sodium chloride in sterile potassium phosphate buffer was prepared by solubilizing 100 mg of monobasic potassium phosphate, 378 mg of potassium dibasic phosphate and 7.8 g of sodium chloride in water. The volume of the diluents solution was brought to 1 liter and the pH was controlled and regulated in the range 7-7.4 (osmolality 250-260 mOsmol/kg). The solution was distributed in vials then sterilized at 121 °C, 1 atm for 20 minutes.

Step (c) Ten ml of diluents solution were used to reconstitute the mixture of sodium ascorbate and sodium chloride.

Cosmetic Treatment: 5 ml of reconstituted solutions were given by subcutaneous injection into local areas with localized cellulite; lipolytic effect, adverse reactions and necrosis were evaluated by histological analysis.

### Clinical study

The cosmetic treatment using the composition according to the invention was administered to a group of subjects who were well informed of the procedure; all gave their consent to the use of the results of the study for obtaining a patent grant.

The selection of subjects enrolled in the study was conducted on the basis of a careful patient analysis excluding the subjects carrying pathological conditions. The attention of the examiners who questioned the group of patients focused mainly on physiological anamnesis. Questions have been asked to clarify: period of menarche, menstrual and related disorders, previous progesterogen/estrogen intake, number and duration of pregnancy, abortion episodes, menopausal age, menopausal symptoms. The evolution of body weight and its variations, whether spontaneous or induced by diet, has been investigated. Particular attention has been drawn to lifestyle, with questions about present and past dietary habits, cigarette smoking, alcohol consumption, and psychoactive substances, physical, current and past exercise, type of work, sexual relations. Finally, without specific tests, attempts have been made to obtain information on psychologic equilibrium, by questions about sleep quality, concentration ability, and mental faculties. The patient's objective examination was conducted according to the established rules of the medical semeiotic routine.

A more specific and in-depth observation of the lower limbs was first made to assess, visually, the presence, location and extent of the irregularities of skin surface typical of cellulite. The examination was performed on patients standing upright: front, posterior and both sides, with particular attention to the femoro-trocanteric, gluteal, super-lateral, super-medial and posterior regions of the thighs and knees, and abdominal, overpubular and suboscapular areas. In subjects apparently exempted from cellulites, the pinch-test was performed, to highlight still subclinical conditions of excessive tension of the subcutaneous interlobular septa. Loco regional palpation has been used to detect the presence of hypodermic nodules and to evaluate their painfulness. By photographic, thermographic and ultrasound instrumental investigations, the condition of each subject have been determined and classified according to a cellulite evaluation scale, *ad hoc* elaborated for the present study:
- grade 0: exempt subjects;
- grade 1: local skin rupture, characterized by few presence of irregular skin surface (madras skin or mattres phenomenon) with pit shaped introflexions;
- grade 2: generalized skin rupture (skin roughness or orange peel skin).

Attention has been paid to skin stretch marks (striae distensae), whose pathogenesis, according to some authors, would have points of contact with cellulite. Particular attention has been paid to searching for symptoms and signs indicative of veno-lymphatic circulation conditions. Subjective (sense of heaviness, paresthesia, diurnal and/or nocturnal cramps) and objective traits have been investigated: first of all, the color of the skin: blotches of excessive pallor, indications of reduced local circulation, presence of alternating irregularly shaped skin areas with different chromatic tone due to local circulation imbalance, hyperchromatic skin due to hemodynamic pigmentation. The presence of areas of atrophy of skin and cutaneous annexes and/or stasis ulcers, the positivity of the fovea sign (declining edema index), and the development of ectasie of small caliber superficial veins (teleangectasie) and varicose dilations of the major vein trunks, the latter classified depending on the severity of the alteration according to the CEAP classification (Clinical-Etiology-Anatomopathy Physiology).

Subcutaneous ultrasound examination was performed by Esaote My Lab ultrasound and 12 MHz linear probe (Esaote S.p.a. Genova).

The biological samples obtained from the subjects who participated in the study were analyzed in order to evaluate:
- routine hematochemical parameters: complete emocromium, leukocyte formula, transaminase, gamma GT, alkaline phosphatase, creatinine, glycemia, azotemia, total cholesterol, HDL cholesterol, LDL cholesterol, triglycerides, whole and fractional bilirubin, complete urine examination.
- hormonal profile: plasmatic 17-β-estradiol, FSH, LH, progesterone, prolactin, FT3, FT4, TSH. Because sex steroids and gonadotrophins are differently produced depending on female ovarian cycle in the fertile age, three separate blood specimens collection have been performed at different dates from subject to subject so as to fall each, respectively in the follicular, ovulatory and luteinic phases. A single blood draw was, however, sufficient for menopausal women.
- main adipokines: the plasma level of leptin, adiponectin and resistin was evaluated.

Finally, the histological analysis of the adipose tissue of the subjects enrolled in the study, taken at various times after the administration of the treatment, was carried out on excision cutaneous biopsies using well-known laboratory techniques to the experts in the field.

### Results

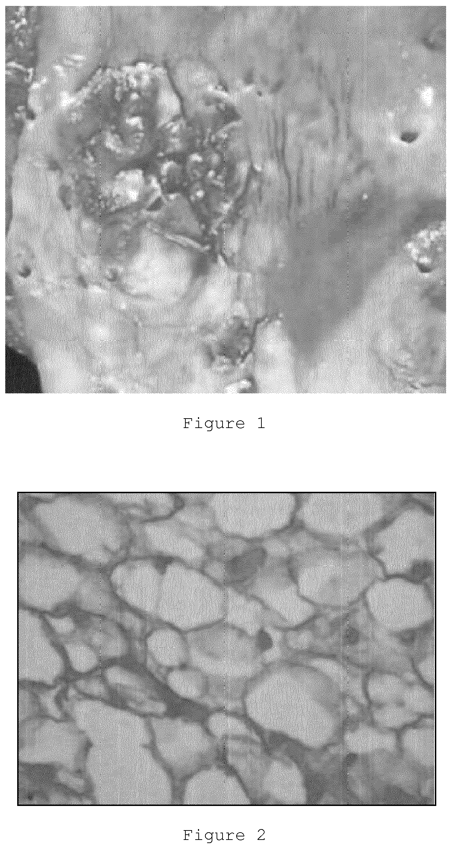

Figure 1 shows the effect of administration of 0.24% sodium ascorbate (12mM) solution, isotonic sodium chloride in phosphate buffer, causing strong on-site inflammation with clear signs of necrosis, an unexpected toxicity. The result has shown that to induce lipolysis without causing any inflammatory effect, concentrations of sodium ascorbate should be less than 0.24%.

Figure 2 shows the reaction in adipose tissue 2 hours after administration of 0.24% sodium ascorbate solution (12 mM), isotonic sodium chloride, with evident haemorrhagic effusion and necrosis.

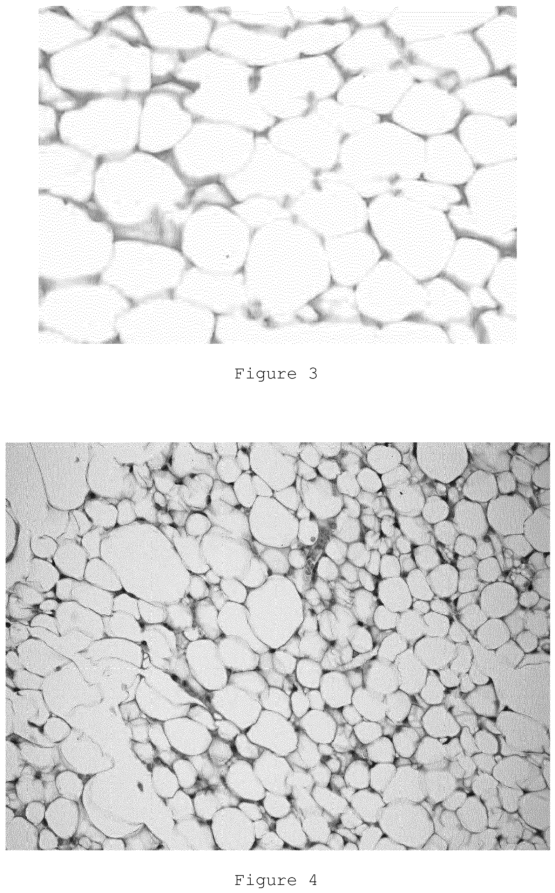

Conversely, the use of 0.12% solution of sodium ascorbate (6mM), 0.45% sodium chloride did not have any lipolytic effect. Administration of 5 ml of the composition was non-toxic, but not effective, even after 1 week as shown in Figure 3.

Figure 4 shows the outcome of minimal lipolysis, with no signs of toxicity and macrophage infiltration one week after administration of 5ml of 0.20% sodium ascorbate (10mM), 0.45% sodium chloride by weight. The image shows small haemorrhagic effusion and mild lipolysis in the lower left part.

Therefore, the study has allowed the development of three buffered, isotonic, ascorbate formulations that injected into the fatty tissue yield no toxicity but with no apparent efficacy (sodium ascorbate 0.12%, 0.15%, 0.17%); the composition comprising 0.20% sodium ascorbate showed a slight adipolysis capacity, but certainly without any efficacy perspective when applied in a true cosmetic treatment for cellulite removal. In this sense, the results were considered to be disappointing and contrary to what is stated in literature or, worse, to what is often practiced as the composition comprising 0.24% by weight of sodium ascorbate has caused a very strong necrosis.

### Example 3 - Identification of active formulations containing the association of ascorbic acid salt and surfactants.

A. Considering the mild adipolysis activity of 0,20% (10 mM) sodium ascorbate solution, a surfactant has been added to said formulation in order to allow better availability and diffusion of sodium ascorbate in the fatty tissue, and thereby, enhance, if possible, cellulite removal effect.

Three surfactants having different hydrophilic/lipophilic ratio (HLB - Idrophilic Lipophilc Balance) have been identified:

| | |
|---|---|
| Glyceryl monooleate | HLB: 2-3 |
| Polysorbate 80 | HLB: 10-12 |
| Ascorbyl Palmitate | HLB: 4-5 |

Three diluent buffering solutions were then prepared, each containing one of the identified surfactants. The three solutions were added to the lyophilized mixture of sodium ascorbate and sodium chloride so as to obtain the following final compositions:
1) 0.20% sodium ascorbate, 0.024%, glyceryl monooleate,
2) 0.20% sodium ascorbate, 0.040% polysorbate 80;
3) 0.20% sodium ascorbate, 0.020% ascorbyl palmitate.

The efficacy of the three compositions was tested on three experimental patient groups following the experimental protocol described in Example 2.

### Results

The experimental study was carried out to evaluate the effect of the addition of the three surfactants to the sodium ascorbate composition.

The formulations containing glyceryl monooleate and polysorbate 80 in addition to 0.20% sodium ascorbate showed a lipolitic activity comparable to that of the composition containing only 10 mM sodium ascorbate (0.20%), so in these two cases, addition of surfactant to the 10mM (0.20%) sodium ascorbate composition did not improve the ability to remove cellulite.

Adversely, the composition containing the association of 0.20% sodium ascorbate and 0.020% ascorbyl palmitate showed activity higher than the composition containing only 0.20% sodium ascorbate.

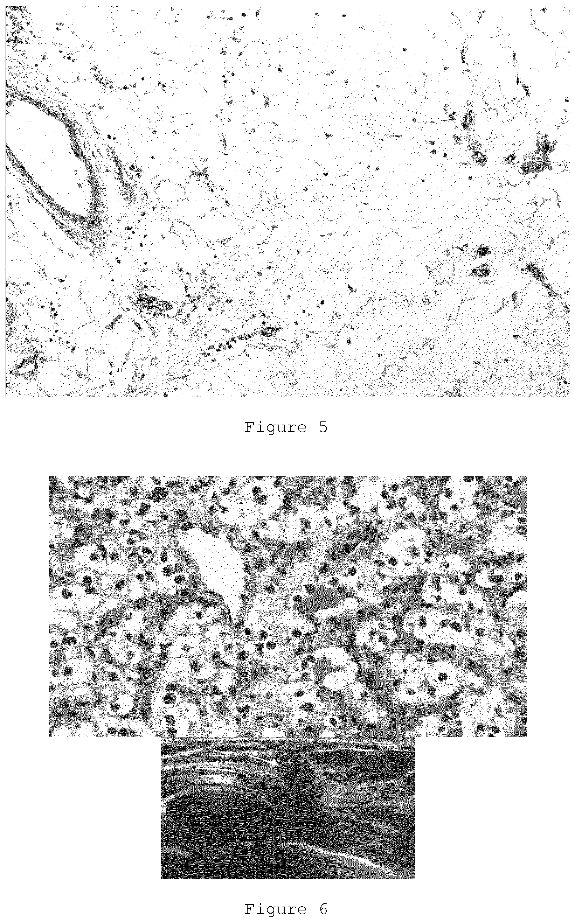

The effect of the administration of the composition 0.20% sodium ascorbate, 0.020% ascorbyl palmitate is shown in Figure 5, wherein 1 week after inoculation signs of vasculitis of small vessels and inflammatory cells between lysed adipocytes are evident; however, such lipolytic activity is associated with the total absence of toxicity and necrosis signs.

Therefore, the result clearly and unambiguously demonstrates that the use of a buffered, isotonic solution containing sodium ascorbate not necessarily is toxic, the presence of surfactants does not change this framework, solutions remain non-toxic at the final concentration of 0.20% sodium ascorbate (10 mM).

In the presence of a lipophilic derivative of ascorbic acid, which is substituted with palmitic acid (ascorbyl palmitate), the composition acquires apparent adipolytic efficacy without any toxicity, as if the surfactant, ascorbyl palmitate, made the formulation particularly biocompatible, or with the ability to modulate the cellulite removal. This ability could be attributed to the lipophilic tail formed by palmitic acid, and hence to its structure.

Observation, however, is not obvious since the glycerol monooleate, although having a lipophilic tail, constituted by oleic acid, structurally similar to palmitic acid, does not have the same effect as the ascorbyl palmitate, in fact the composition with 0.20% sodium ascorbate and glycerol monooleate was found to be ineffective.

B. In order to verify the effect of adding the surfactant to the 0.24% by weight composition of sodium ascorbate in the possible reduction of necrosis, the following compositions were prepared and tested:
4) 0.24% sodium ascorbate, 0.024%, glyceryl monooleate,
5) 0.24% sodium ascorbate, 0.040% polysorbate 80;
6) 0.24% sodium ascorbate, 0.020% palmitate ascorbyl.

### Results

Administration of 0.24% sodium ascorbate, 0.024%, glyceryl monooleate composition, has caused a remarkable necrotic effect as shown in Figure 6, wherein 7 days after administration necrotic agglomerates with major macrophage infiltration and bleeding are evident. In this case, despite the addition of the surfactant, the inflammatory and necrotic effect induced by 0.24% sodium ascorbate is confirmed; glyceryl monooleate is able to, neither modulate, nor eliminate the necrotic effect.

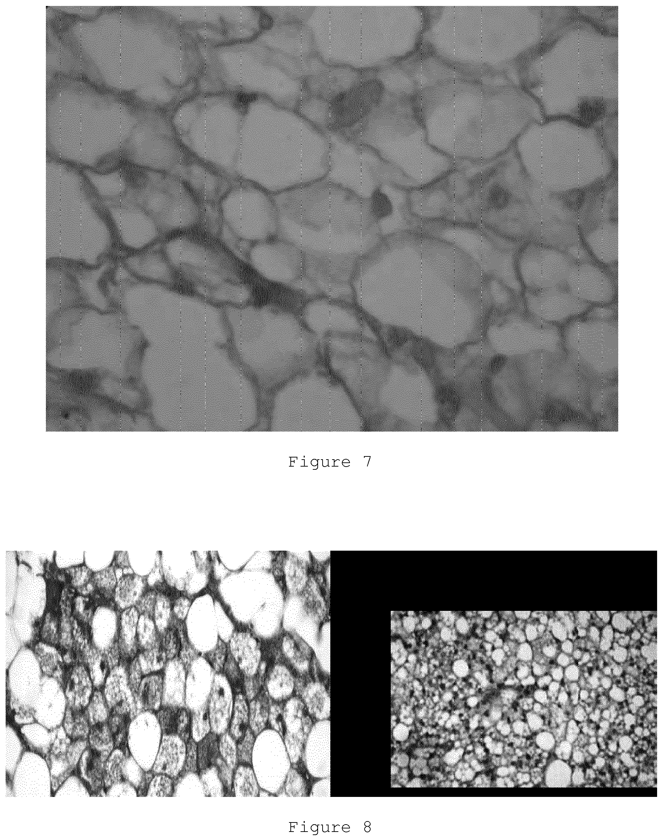

Very accentuated necrosis also occurs when administering the composition containing 0.24% by weight of sodium ascorbate, 0.040% by weight of polysorbate 80, shown in Figure 7. Histological analysis confirms the adipose tissue reaction 2 hours after composition administration which shows evident presence of extravasal effusion.

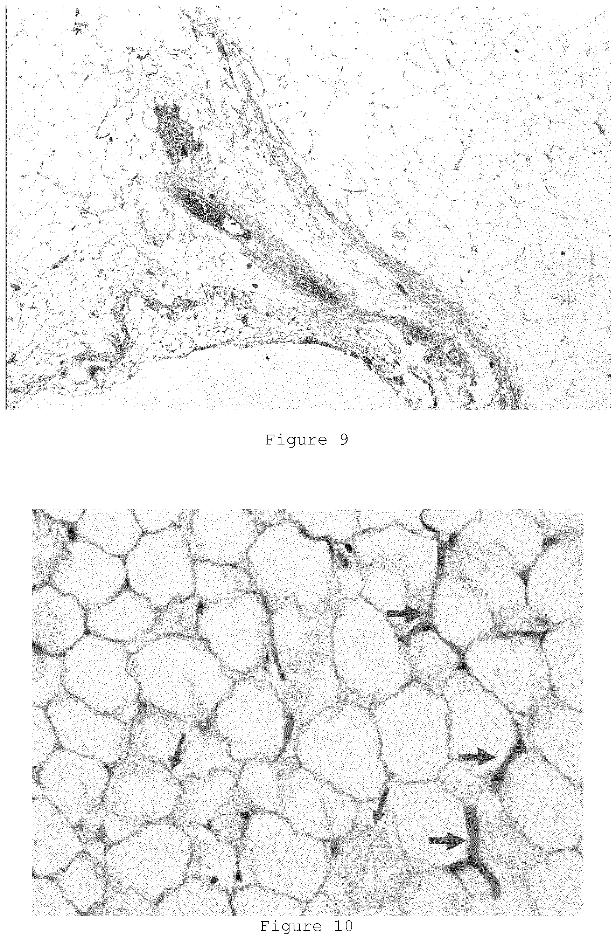

The result of histological analysis two days after administration of the composition containing 0.24% by weight of sodium ascorbate, 0.040% by weight of polysorbate 80 is illustrated in Figure 8 showing clear inflammatory tissue rich in macrophages and cleavage of some fatty cells; also in this case the inflammatory and necrotic effect induced by 0,24% sodium ascorbate is confirmed, and polysorbate 80 can neither modify nor eliminate the necrotic effect. Surprisingly, the buffered isotonic composition comprising 0.24% by weight of sodium ascorbate, 0.020% palmitate ascorbyl, has demonstrated to have adipolytic and lipoclasic effect, since histology showed a decrease in the number of adipocytes, without any inflammation or necrosis signs. In contrast to the necrosis caused by the composition used to reduce cellulite blemishes comprising 0.24% sodium ascorbate, as illustrated in Figure 9, showing the histological analysis of adipose tissue after administration of 0.24% sodium ascorbate, 0.020% ascorbyl palmitate composition according to the invention, pointing out vasculitis and vascular congestion signs with well-defined lysed adipocytes and signs of lipoemulsion.

Figures 10 to 14 show the timing of occurrence of technical effect caused by the administration of the composition according to the invention, figures show that the adipolytic/lipoclasic effect is prolonged for a period of 23 to 28 days, ensuring reduction of cellulitis without inflammation or necrosis, as shown by the histological analysis results. Figure 10 shows histologic analysis of adipose tissue 5 days after the administration of 0.24% by weight sodium ascorbate, 0.020% by weight ascorbyl palmitate composition which highlights vascularization with neoformed little vases surrounding adipocytes (red arrow), magnified, but pale, nuclei and vacuoles inside, resulting from a possible reaction to an insult, or due to a repair effect, however, nucleoli are not evident. The adipocytes exhibit a stained cytoplasm.

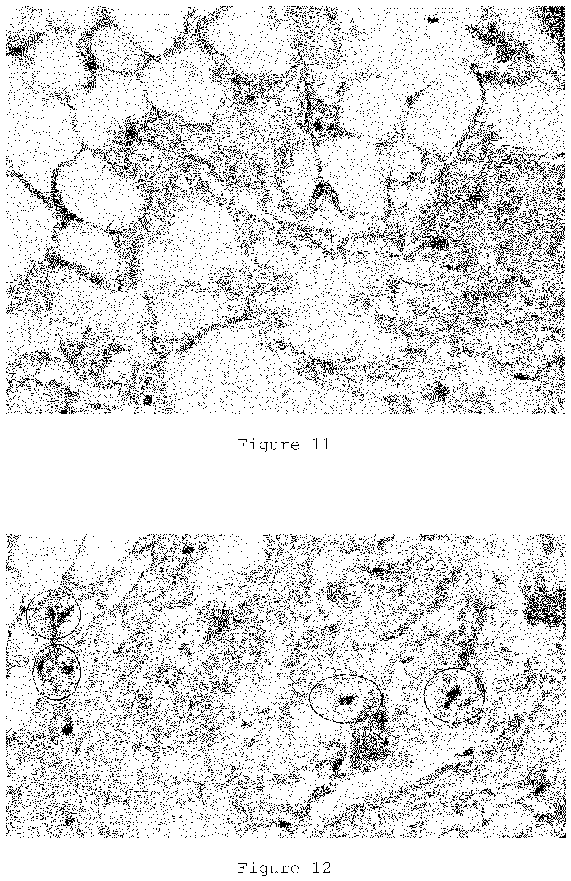

Figure 11 shows the histological analysis of adipose tissue ten days after administration of 0.24% by weight of sodium ascorbate, 0.020% by weight of ascorbyl palmitate composition, wherein alterations already evident 5 days after administration are even more visible. Further adipocytes fragmentation is associated with a granular background.

Histological analysis carried out 15 days after administration of 0.24% sodium ascorbate, 0.020% ascorbyl palmitate composition shown in Figure 12 highlights residual structures of adipocytes with aggregates of apoptotic products and very dark cell nuclei.

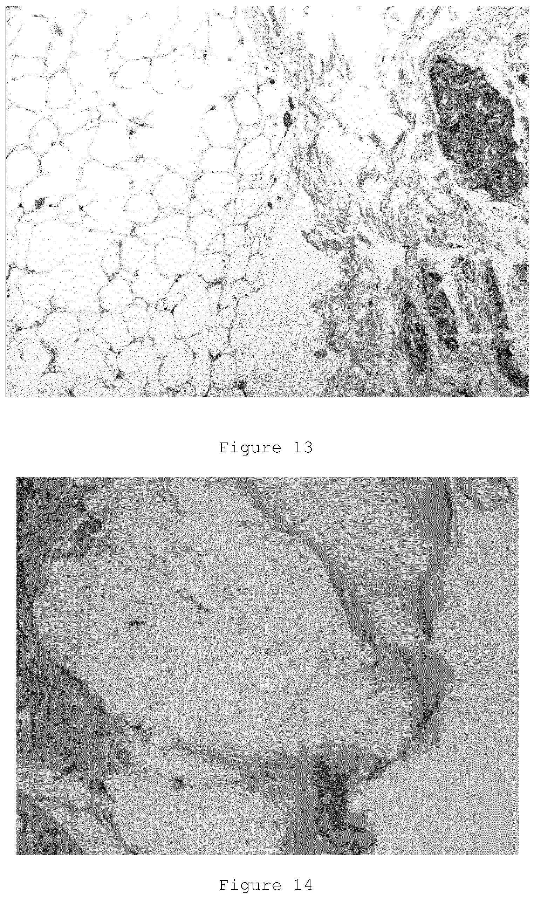

The technical effect of the treatment with the composition object of the invention 0.24% sodium ascorbate, 0.020% ascorbyl palmitate 23 days after the first administration of the composition is shown in Figure 13, wherein collagene with giant cell granuloma and lipolysis region of fatty cells with lipoemulsion are observed.

Finally, Figure 14 shows areas with evident tissue lipoclasis 25 days after the first administration of the composition.

### Conclusion

The results of the experimental study demonstrate that the undoubted lipoclasic effect obtained by the use of association of sodium ascorbate and a lipophilic derivative of ascorbic acid, which have been substituted by either palmitic acid or stearic acid, or tetraesyl diacyl ascorbate, is a complex phenomenon in which the presence of the lipophilic derivative of ascorbic acid exerts an action enhancing and modulating the phenomenon itself. This activity could be mainly attributed to the presence of lipophilic tail/s of ascorbic acid derivatives. This result is surprising and unexpected compared to the foreseeable on the basis of the observation obtained in the experiment, and to what is known by the state of the art, wherein the composition comprising only sodium ascorbate at the highest concentration of 0.24% is used. According to the present invention, the total concentration of the ascorbate ion is 0.2204%, resulting from the sum of the concentration of the ascorbate ion in solution of 0.24% sodium ascorbate (0.212%) and 0.0084%, equal to the concentration of ascorbate ion derived from 0.02% ascorbyl palmitate, or other lipophilic derivative of ascorbic acid variedly substituted. Therefore, the increase in the amount of ascorbate anion, resulting from the lipophilic derivative component of ascorbic acid present in the combination, although limited, does not contribute to increase the necrotic phenomenon; however, it has a lipoclasic effect, with no necrosis, although having a final concentration of the higher ascorbate ion.

Surprisingly, the presence of the lipophilic component is able to increase the effectiveness of the composition and to modify the lipoclasic phenomenon, demonstrating that the removal of cellulite is not exclusively due to the pro-oxidant capacity of the ascorbate anion.

## Claims

1. Aqueous, isotonic and buffered composition for topical and subcutaneous use, **characterized in that** it comprises a salt of ascorbic acid and ascorbyl palmitate, sodium chloride and buffering system,
wherein the salt of ascorbic acid and ascorbic palmitate are respectively in a ratio of 12:1 by weight, and wherein said salt of ascorbic acid is in a concentration ranging between 0,12 and 0,25% by the total weight and the ascorbyl palmitate is in a concentration ranging between 0,01 and 0,03% by total weight, for use as a lipolytic/lipoclasic agent in an anti-cellulite cosmetic treatment.

2. The composition according to claim 1 wherein the salt is a sodium, lithium, potassium, magnesium, calcium salt.

3. The composition according to claims 1 and 2 having pH ranging between 7.0 and 7.4 and osmolality between 250 and 340 mOsm/Kg.

4. The composition according to anyone of claims from 1 to 3 further comprising: solvents, excipients, acceptable diluents and carriers allowing administration by subcutaneous injection.

5. Aqueous, isotonic and buffered composition for topical and subcutaneous use according to claim 1, having the following formulation:
| | | |
|---|---|---|
| Sodium ascorbate | 0.24% | w/w |
| Ascorbyl palmitate | 0.020% | w/w |
| Sodium chloride | 0.96% | w/w |
| Potassium phosphate dibasic (K₂HPO₄) | 0.0378% | w/w |
| Potassium phosphate monobasic (KH₂PO₄) | 0.010% | w/w |
| distilled water | q. s. to 100 | |

6. Non therapeutic, cosmetic use of an aqueous, isotonic and buffered composition as defined in the preceding claims, for anti-cellulite treatment comprising administering through subcutaneous injection to a subject in need of treatment an effective amount of composition comprising 0.12 to 0.25 % by total weight of salt of ascorbic acid and 0.01 to 0.03% by total weight of the lipophilic derivative of ascorbic acid,
wherein the effective dose of subcutaneously administered composition is equal to 5 ml, useful for treating a body surface area equal to 10-20 cm².

7. Kit comprising:
a) at least one dark glass vial of 10 mL volume containing the mixture of freeze-dried powders composed of sterile ascorbic acid salt, phosphate buffer and sodium chloride, in amount necessary for the topical administration of one dose of the final reconstituted composition as defined in anyone of claims 1 and 5;
b) at least one vial of 5 ml volume containing 5 ml of sterile diluent comprising the lipophilic derivative of ascorbic acid substituted with palmitic acid or stearic acid, necessary to reconstitute the amount of mixture in (a) for the topical administration of one dose of the final reconstituted composition as defined in anyone of claims 1 and 5.

## Patentansprüche

1. Wässrige, isotonische und gepufferte Zusammensetzung zur topischen und subkutanen Anwendung, **dadurch gekennzeichnet, dass** sie ein Ascorbinsäuresalz und Ascorbylpalmitat, Natriumchlorid und Puffersystem umfasst,
wobei das Ascorbinsäuresalz und das Ascorbinsäurepalmitat jeweils in einem Gewichtsverhältnis von 12:1 vorliegen und wobei das Ascorbinsäuresalz in einer Konzentration im Bereich zwischen 0,12 und 0,25 Gew.-% des Gesamtgewichts vorliegt und das Ascorbylpalmitat in einer Konzentration zwischen 0,01 und 0,03 Gew.-% zur Verwendung als lipolytisches/lipoklasisches Mittel bei einer kosmetischen Behandlung gegen Cellulite vorliegt.

2. Zusammensetzung nach Anspruch 1, wobei das Salz ein Natrium-, Lithium-, Kalium-, Magnesium-, Calciumsalz ist.

3. Zusammensetzung nach Anspruch 1 und 2 mit einem pH-Wert im Bereich zwischen 7,0 und 7,4 und einer Osmolalität zwischen 250 und 340 mOsm/kg.

4. Zusammensetzung nach einem der Ansprüche 1 bis 3, ferner umfassend: Lösungsmittel, Hilfsstoffe, akzeptable Verdünnungsmittel und Träger, die eine Verabreichung durch subkutane Injektion ermöglichen.

5. Wässrige, isotonische und gepufferte Zusammensetzung zur topischen und subkutanen Verwendung nach Anspruch 1 mit der folgenden Formulierung:
| | |
|---|---|
| Natriumascorbat | 0,24 Gew.-% |
| Ascorbylpalmitat | 0,020 Gew.-% |
| Natriumchlorid | 0,96 Gew.-% |
| Kaliumphosphat zweibasisch (K₂HPO₄) | 0,0378 Gew.-% |
| Kaliumphosphat einbasig (KH₂PO₄) | 0,010 Gew.-% |
| destilliertes Wasser | q. s. bis 100. |

6. Nichttherapeutische, kosmetische Verwendung einer wässrigen, isotonischen und gepufferten Zusammensetzung nach den vorhergehenden Ansprüchen zur Behandlung gegen Cellulite, umfassend das Verabreichen einer wirksamen Menge der Zusammensetzung, die 0,12 bis 0,25 Gew.-% Ascorbinsäuresalz und 0,01 bis 0,03 Gew.-% des lipophilen Ascorbinsäurederivats umfasst, durch subkutane Injektion an ein behandlungsbedürftiges Subjekt,
wobei die wirksame Dosis der subkutan verabreichten Zusammensetzung 5 ml beträgt, was zur Behandlung einer Körperoberfläche von 10 bis 20 cm² nützlich ist.

7. Kit, umfassend:
a) mindestens ein dunkles Glasfläschchen mit einem Volumen von 10 ml, das die Mischung aus gefriergetrockneten Pulvern, die aus sterilem Ascorbinsäuresalz, Phosphatpuffer und Natriumchlorid zusammengesetzt sind, in einer Menge enthält, die für die topische Verabreichung einer Dosis der endgültigen rekonstituierten Zusammensetzung nach einem der Ansprüche 1 und 5 notwendig ist;
b) mindestens ein Fläschchen mit einem Volumen von 5 ml, das 5 ml steriles Verdünnungsmittel umfassend das das mit Palmitinsäure oder Stearinsäure substituierte lipophile Ascorbinsäurederivat enthält, die notwendig sind, um die Menge der Mischung in (a) für die topische Verabreichung einer Dosis der endgültigen rekonstituierten Zusammensetzung nach einem der Ansprüche 1 und 5 zu rekonstituieren.

## Revendications

1. Composition aqueuse, isotonique et tamponnée pour une utilisation topique et sous-cutanée, **caractérisée en ce qu'**elle comprend un sel d'acide ascorbique et du palmitate d'ascorbyle, du chlorure de sodium et un système tampon,
dans laquelle le sel d'acide ascorbique et le palmitate ascorbique sont respectivement présents en un rapport de 12/1 en poids, et dans laquelle ledit sel d'acide ascorbique est présent en une concentration dans la plage de 0,12 à 0,25 % en poids total et le palmitate d'ascorbyle est présent en une concentration dans la plage comprise entre 0,01 et 0,03% en poids total, pour son utilisation en tant qu'agent lipolytique/lipoclasique dans un traitement cosmétique anticellulite.

2. Composition selon la revendication 1, dans laquelle le sel est un sel de sodium, de lithium, de potassium, de magnésium, de calcium.

3. Composition selon les revendications 1 et 2 ayant un pH dans la plage de 7,0 à 7,4 et une osmolalité entre 250 et 340 mOsm/kg.

4. Composition selon l'une quelconque des revendications 1 à 3 comprenant en outre : des solvants, des excipients, des diluants acceptables et des vecteurs permettant l'administration par injection sous-cutanée.

5. Composition aqueuse, isotonique et tamponnée pour une utilisation topique et sous-cutanée selon la revendication 1, ayant la formulation suivante :
| | |
|---|---|
| ascorbate de sodium | 0,24 % p/p |
| palmitate d'ascorbyle | 0,020 % p/p |
| chlorure de sodium | 0,96 % p/p |
| phosphate de potassium dibasique (KH₂PO₄) | 0,0378 % p/p |
| phosphate de potassium monobasique (KH₂HPO₄) | 0,010 % p/p |
| eau distillée | q.s. jusqu'à 100 |

6. Utilisation cosmétique, non thérapeutique d'une composition aqueuse isotonique et tamponnée telle que définie dans les revendications précédentes pour un traitement anticellulite comprenant l'administration par une injection sous-cutanée à un sujet nécessitant un traitement d'une quantité efficace d'une composition comprenant de 0,12 à 0,25 % en poids total de sel d'acide ascorbique et de 0,01 à 0,03 % en poids total de dérivé lipophile d'acide ascorbique,
dans laquelle la dose efficace de composition administrée par voie sous-cutanée est égale à 5 ml, utile pour un traitement d'une surface corporelle égale à 10 à 20 cm².

7. Kit comprenant :
a) au moins un flacon en verre foncé d'un volume de 10 ml contenant le mélange de poudres lyophilisées composé d'un sel d'acide ascorbique stérile, d'un tampon phosphate et de chlorure de sodium, en une quantité nécessaire pour l'administration topique d'une dose de la composition reconstituée finale telle que définie dans l'une quelconque des revendications 1 et 5 ;
b) au moins un flacon d'un volume de 5 ml contenant 5 ml de diluant stérile comprenant le dérivé lipophile d'acide ascorbique substitué avec de l'acide palmitique ou de l'acide stéarique, nécessaire pour reconstituer la quantité de mélange dans (a) pour l'administration topique d'une dose de la composition finale reconstituée telle que définie dans l'une quelconque des revendications 1 et 5.
